# EUROPEAN PATENT SPECIFICATION

(11) **EP 1 742 685 B1**
(45) Date of publication and mention of the grant of the patent: **16.10.2013**
(21) Application number: 05718922.7
(22) Date of filing: 28.03.2005
(51) Int. Cl.: A61M 5/20, A61M 5/145

(54) **SYRINGE PUMP**
SPRITZENPUMPE
POUSSE-SERINGUE

(30) Priority: 29.03.2004 US 810686
(43) Date of publication of application: 17.01.2007
(73) Proprietor: Fertiligent Ltd., 10550 Migdal HaEmek (IL)
(72) Inventor: KLEIN, Ronnie, 34750 Haifa (IL); WEICHSELBAUM, Amnon, 32207 Haifa (IL)
(74) Representative: Dennemeyer & Associates S.A.
(86) International application number: PCT/IL2005/000349
(87) International publication number: WO 2005/092409

(56) References cited:
- GB-A- 1 026 593
- US-A- 2 605 765
- US-A- 5 788 673
- US-A1- 2002 111 589
- US-A1- 2004 019 325
- US-A1- 2004 059 186

## Description

### FIELD OF THE INVENTION

The present invention relates generally to syringe pumps, and particularly to a disposable syringe pump for sperm delivery, such as in slow-release insemination.

### BACKGROUND OF THE INVENTION

Microfluidic pumping devices are used in numerous applications, such as administration of medicine and biological and pharmaceutical research. Such pumping devices include mechanical pumps, such as syringe-type pumps and micromechanical pumps, and non-mechanical pumps, such as electrohydrodynamic pumps, electro-osmotic flow pumps, electrowetting pumps, and thermocapillary pumps.

There are drawbacks to different pumping devices. For example, a steady flow rate is difficult to achieve. Moreover, many mechanical pumps require an electrical power source, as do pumps that operate based on electrical properties. Many of these pumps are costly and often have slow response times.

Conventional syringe pumps are typically employed with either a syringe or a vial and plunger system for administering a liquid to a patient. In such conventional systems, a syringe or vial of the liquid is oriented vertically in a fixed position on the syringe pump. The bottom of the syringe or vial defines a discharge port connected to a flexible, hollow tubing which extends to the patient. The plunger or piston of the apparatus is engaged with the moving pusher plate or drive member of the syringe pump and is driven downwardly into the syringe body or vial to force the liquid agent from the syringe body or vial through the tubing and into the patient.

An example of such a syringe pump is described in a system of PCT published patent application WO03008102 or US 5788673. The system employs a microchannel and a gravity driven pump comprising horizontally oriented fluid supply reservoirs. The pump supplies fluid to the microchannel at a substantially constant rate. The device may be used, among other things, for motile sperm sorting.

### SUMMARY OF THE INVENTION

The present invention seeks to provide a novel, disposable syringe pump for sperm delivery, such as in slow-release insemination, as described more in detail hereinbelow. The invention may have other applications and is not limited just to slow-release insemination. For example, the invention may be used in the laboratory or other research area for pumping sperm and other fluids.

There is thus provided in accordance with an embodiment of the present invention a syringe pump including a syringe including a plunger that slides in a body which has a discharge port, a driving mechanism coupled to the syringe, including a cylinder in which a piston mounted on a shaft slides, and a biasing device operative to apply an urging force on the piston to drive the piston distally in the cylinder, and a safety catch that initially prevents the biasing device from moving the piston, the safety catch being removable to permit the biasing device to move the piston.

The syringe pump may include one or more of the following features. For example, the cylinder may be at least partially filled with a hydraulic fluid. The piston may be formed with a vent hole that passes through the thickness of the piston and may be in fluid communication with a port in the shaft, wherein the vent hole and the port permit flow of the hydraulic fluid from a distal portion of the cylinder in front of the piston to a proximal portion of the cylinder behind the piston. The biasing force of the biasing device on the piston and hydraulic damping of the hydraulic fluid may provide a close-to-linear pumping force.

The driving mechanism may be coupled to a head of the plunger with a clasp. The biasing device may include a coil spring disposed on a portion of the shaft. The syringe and the driving mechanism may be housed in a casing. The casing may have a window through which travel of the driving mechanism may be observable.

### BRIEF DESCRIPTION OF THE DRAWINGS

The present invention will be understood and appreciated more fully from the following detailed description taken in conjunction with the drawings in which:
Fig. 1 is a simplified pictorial illustration of a disposable syringe pump, constructed and operative in accordance with an embodiment of the present invention;

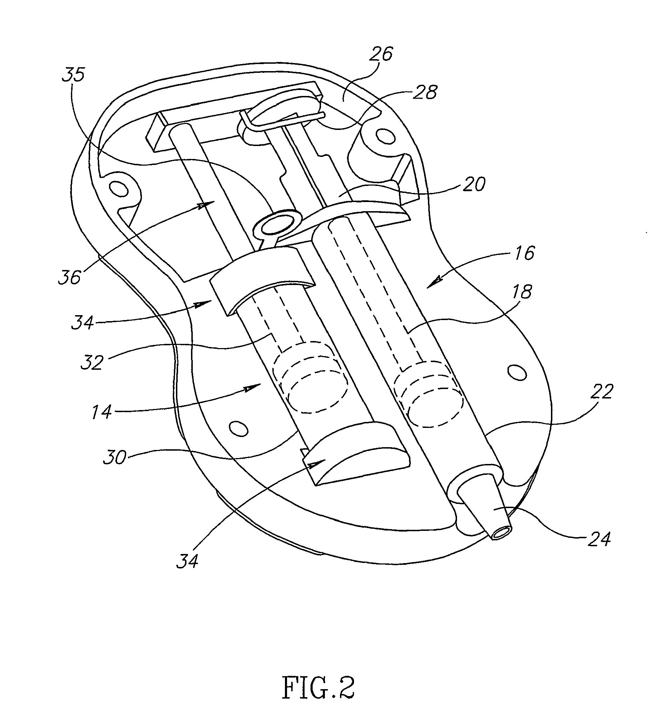

Fig. 2 is a simplified cutaway illustration of the syringe pump of Fig. 1, showing inner components thereof;
Fig. 3 is a simplified cutaway illustration of a driving mechanism used in the syringe pump of Fig. 1, constructed and operative in accordance with an embodiment of the present invention; and
Fig. 4 is a sectional illustration of a plunger used in the driving mechanism of Fig. 3, in accordance with an embodiment of the present invention.

### DETAILED DESCRIPTION OF EMBODIMENTS

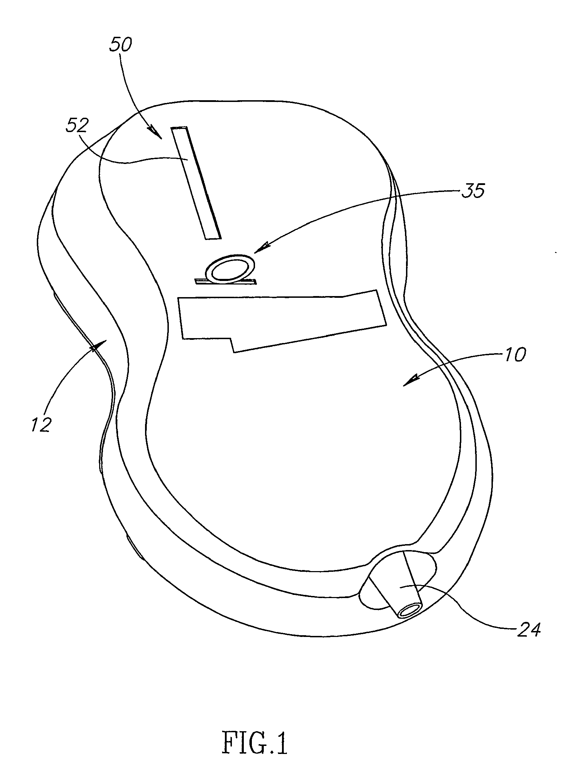

Reference is now made to Fig. 1, which illustrates a syringe pump 10, constructed and operative in accordance with an embodiment of the present invention.

The syringe pump 10 may include an outer casing 12, in which are housed a driving mechanism 14 coupled to a syringe 16. The casing 12 is illustrated as being constructed of two halves joined together (e.g., by screws, bonding, sonic welding or any other suitable method of connection), but may be constructed of one part or many parts as well. The syringe pump 10 may have any size and shape, which may depend, among other things, on the size and shape of the syringe 16 and the required flow rate. In one non-limiting embodiment of the invention, syringe pump 10 may be about 5-9 cm long, 5 cm wide and 3 cm thick.

The syringe 16 may include a body 18 in which a plunger 20 slides. The body 18 may hold any suitable volume of sperm 22, such as but not limited to, about 0.3-1.5 cc. The syringe 16 may have a discharge port 24, which may be connected to suitable tubing and a filter (not shown) for sperm delivery, such as in slow-release insemination. The syringe 16 and its parts may be made of any medically safe material, such as but not limited to, polycarbonate, and may be completely disposable.

The driving mechanism 14 may be coupled to a head 26 of plunger 20, such as by means of a clasp 28 or any other suitable link or connection. The driving mechanism 14 may include a cylinder 30 in which a piston 32 slides. The travel of piston 32 inside cylinder 30 may be bounded by end caps 34. Piston 32 may be mounted on a shaft 36, which is connected to clasp 28.

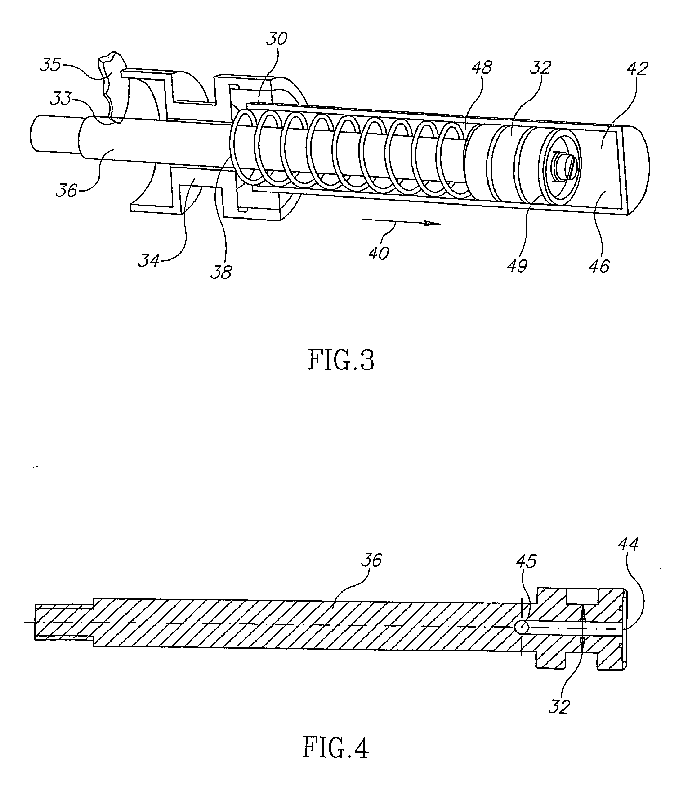

Reference is now made additionally to Figs. 3 and 4. The driving mechanism 14 may include a biasing device 38, such as but not limited to, a coil spring, disposed on a portion of shaft 36 proximal to the piston 32. The biasing device 38 is operative to apply an urging force on piston 32 to drive piston 32 distally (in the direction of an arrow 40) in cylinder 30. Initially, a safety catch 35 may arrest movement of shaft 36 and piston 32. For example, the safety catch 35 may initially abut against one of the end caps 34 and sit in a notch 33 formed in shaft 36, thereby preventing biasing device 38 from expanding and moving piston 32.

Cylinder 30 may be at least partially filled with a hydraulic fluid 42, such as but not limited to, glycerin. Piston 32 may be formed with a relatively tiny vent hole 44 (such as but not limited to, a diameter of 0.1 mm) that passes through the thickness of piston 32 and is in fluid communication with a port 45 in shaft 36 on the proximal side of piston 32 (see Fig. 4). The combination of vent hole 44 and port 45 permit flow of hydraulic fluid 42 from a distal portion 46 of cylinder 30 (that is, in front of piston 32) to a proximal portion 48 of cylinder 30 (that is, behind piston 32) (see Fig. 3). Accordingly, after removal of safety catch 35, biasing device 38 pushes piston 32 distally in the direction of arrow 40, and hydraulic fluid 42 is transferred between the distal portion 46 to the proximal portion 48 of cylinder 30 (located at the posterior end of the moving plunger) via vent hole 44 and port 45. The combination of the biasing force of biasing device 38 and the hydraulic damping of the hydraulic fluid 42 may provide a close-to-linear pumping force.

As seen in Fig. 3, a regulator valve 49 may be disposed in the vent hole 44 that regulates the transfer of hydraulic fluid 42 between the distal portion 46 to the proximal portion 48 of cylinder 30. The regulator valve 49 may include a threaded screw shaft that may be turned (e.g., with a screwdriver) to regulate the size of the opening for passage therethrough of hydraulic fluid 42.

Referring again to Fig. 1, it is seen that the casing 12 may be provided with a window 50 through which the travel and forward progress of driving mechanism 14 may be observed. For example, the widow 50 may expose a tab 52 formed on shaft 36, which easily allows observation of the movement of shaft 36.

The flow or pumping rate of syringe pump 10 may be adjusted by adjusting or selecting different operating parameters, such as but not limited to, the spring coefficient of biasing device 38, sizes and shapes of vent hole 44 and port 45, the position of regulator valve 49, the cross sectional area of cylinder 30 and of body 18, and/or the viscosity of hydraulic fluid 42 (e.g., in the range of 50-1000 centipoise at 20°C).

It is appreciated that various features of the invention which are, for clarity, described in the contexts of separate embodiments, may also be provided in combination in a single embodiment. Conversely, various features of the invention which are, for brevity, described in the context of a single embodiment, may also be provided separately or in any suitable subcombination.

## Claims

1. A syringe pump (10) comprising:
a syringe (16) comprising a plunger (20) that slides in a body (18) which has a discharge port (24);
a driving mechanism (14) coupled to said syringe (16), comprising a cylinder (30) in which a piston (32) mounted on a shaft (36) slides, and a biasing device (38) operative to apply an urging force on said piston (32) to drive said piston (32) distally in said cylinder (30); and
a safety catch (35) that initially prevents said biasing device (38) from moving said piston (32), said safety catch (35) being removable to permit said biasing device (38) to move said piston (32); and
wherein said cylinder (30) is at least partially filled with a hydraulic fluid (42), and said piston (32) is formed with a vent hole (44) that passes through the thickness of said piston (32) and is in fluid communication with a port (45) in said shaft (36), wherein said vent hole (44) and said port (45) permit flow of said hydraulic fluid (42) from a distal portion (46) of said cylinder (30) in front of said piston (32) to a proximal portion (48) of said cylinder (30) behind said piston (32),
**characterised by**
a regulator valve (49) disposed in said vent hole (44) operative to regulate transfer of the hydraulic fluid (42) between the distal portion (46) to the proximal portion (48) of said cylinder (30).

2. The syringe pump (10) according to claim 1, wherein said driving mechanism (14) is coupled to a head (26) of said plunger (20) by means of a clasp (28) connected to said shaft (36), and said syringe (16) contains sperm (22) therein, and said syringe (16) and said driving mechanism (14) are housed in a casing (12), and travel of said piston (32) inside said cylinder (30) is bounded by end caps (34).

3. The syringe pump (10) according to claim 2, wherein said safety catch (35) initially abuts against one of said end caps (34) and sits in a notch (33) formed in said shaft (36), thereby preventing said biasing device (38) from expanding and moving said piston (32).

4. The syringe pump (10) according to claim 1, wherein a biasing force of said biasing device (38) on said piston (32) and a hydraulic damping of said hydraulic fluid (42) provide a close-to-linear pumping force.

5. The syringe pump (10) according to claim 2, wherein said casing (12) has a window (50) through which travel of said driving mechanism (14) is observable.

6. The syringe pump (10) according to claim 5, wherein said window (50) exposes a tab (52) formed on said shaft (36).

## Patentansprüche

1. Spritzenpumpe (10), umfassend:
eine Spritze (16), die einen Kolben (20) umfasst, der in einem Körper (18) gleitet, der eine Ausgabeöffnung (24) aufweist;
einen mit der Spritze (16) verbundenen Antriebsmechanismus (14), der einen Zylinder (30), in dem ein auf einer Welle (36) angebrachter Kolben (32) gleitet, und eine Vorspannvorrichtung (38) umfasst, die betreibbar ist, um eine Drängkraft auf den Kolben (32) anzulegen, um den Kolben (32) im Zylinder (30) distal zu treiben; und
einen Sicherungsbügel (35), der zunächst verhindert, dass die Vorspannvorrichtung (38) den Kolben (32) bewegt, wobei der Sicherungsbügel (35) entfernbar ist, um zu ermöglichen, dass die Vorspannvorrichtung (38) den Kolben (32) bewegt; und
wobei der Zylinder (30) zumindest teilweise mit einem hydraulischen Fluid (42) gefüllt ist, und wobei der Kolben (32) mit einem Lüftungsloch (44) ausgebildet ist, das durch die Dicke des Kolbens (32) verläuft und in Fluidverbindung mit einer Öffnung (45) in der Welle (36) steht, wobei das Lüftungsloch (44) und die Öffnung (45) ein Fließen des hydraulischen Fluids (42) von einem distalen Abschnitt (46) des Zylinders (30) vor dem Kolben (32) in einen proximalen Abschnitt (48) des Zylinders (30) hinter dem Kolben (32) ermöglicht, **gekennzeichnet durch**
ein Regelventil (49), das im Lüftungsloch (44) angeordnet und betreibbar ist, um die Übertragung des hydraulischen Fluids (42) vom distalen Abschnitt (46) zum proximalen Abschnitt (48) des Zylinders (30) zu regulieren.

2. Spritzenpumpe (10) nach Anspruch 1, wobei der Antriebsmechanismus (14) über eine mit der Welle (36) verbundene Schnalle (28) mit einem Kopf (26) des Kolbens (20) verbunden ist, und wobei die Spritze (16) Sperma (22) enthält, und wobei die Spritze (16) und der Antriebsmechanismus (14) in einem Gehäuse (12) aufgenommen sind, und wobei die Bewegung des Kolbens (32) innerhalb des Zylinders (30) durch Endkappen (34) begrenzt ist.

3. Spritzenpumpe (10) nach Anspruch 2, wobei der Sicherungsbügel (35) zunächst an einer der Endkappen (34) anliegt und in einer in der Welle (36) gebildeten Kerbe (33) sitzt, wodurch verhindert wird, dass die Vorspannvorrichtung (38) den Kolben (32) erweitert und bewegt.

4. Spritzenpumpe (10) nach Anspruch 1, wobei eine Vorspannkraft der Vorspannvorrichtung (38) auf den Kolben (32) und eine hydraulische Dämpfung des hydraulischen Fluids (42) eine beinahe lineare Pumpkraft bereitstellen.

5. Spritzenpumpe (10) nach Anspruch 2, wobei das Gehäuse (12) ein Fenster (50) aufweist, durch das die Bewegung des Antriebsmechanismus (14) beobachtbar ist.

6. Spritzenpumpe (10) nach Anspruch 5, wobei das Fenster (50) einen auf der Welle (36) gebildeten Streifen (52) freilegt.

## Revendications

1. Pousse-seringue (10) comprenant:
une seringue (16) comprenant un piston plongeur (20) qui coulisse dans un corps (18) qui a un orifice de décharge (24);
un mécanisme d'entraînement (14) couplé à ladite seringue (16), comprenant un cylindre (30) dans lequel coulisse un piston (32) monté sur une tige (36), et un dispositif de sollicitation (38) opérationnel pour appliquer une force de poussée sur ledit piston (32) afin d'entraîner ledit piston (32) de manière distale dans ledit cylindre (30); et
un cran de sécurité (35) qui empêche initialement ledit dispositif de sollicitation (38) de déplacer ledit piston (32), ledit cran de sécurité (35) étant amovible pour permettre audit dispositif de sollicitation (38) de déplacer ledit piston (32); et
dans lequel ledit cylindre (30) est au moins partiellement rempli avec un fluide hydraulique (42), et ledit piston (32) est formé avec un trou d'évent (44) qui traverse l'épaisseur dudit piston (32) et est en communication de fluide avec un orifice (45) dans ladite tige (36), dans lequel ledit trou d'évent (44) et ledit orifice (45) permettent l'écoulement dudit fluide hydraulique (42) depuis une partie distale (46) dudit cylindre (30) en face dudit piston (32) vers une partie proximale (48) dudit cylindre (30) derrière ledit piston (32),
**caractérisé par**:
un manodétendeur (49) disposé dans ledit trou d'évent (44) opérationnel pour réguler le transfert du fluide hydraulique (42) entre la partie distale (46) et la partie proximale (48) dudit cylindre (30).

2. Pousse-seringue (10) selon la revendication 1, dans lequel ledit mécanisme d'entraînement (14) est couplé à une tête (26) dudit piston plongeur (20) au moyen d'un crochet (28) raccordé à ladite tige (36) et ladite seringue (16) contient du sperme (22) à l'intérieur de cette dernière, et ladite seringue (16) et ledit mécanisme d'entraînement (14) sont logés dans un boîtier (12) et le déplacement dudit piston (32) à l'intérieur dudit cylindre (30) est délimité par des capuchons d'extrémité (34).

3. Pousse-seringue (10) selon la revendication 2, dans lequel ledit cran de sécurité (35) vient initialement en butée contre l'un desdits capuchons d'extrémité (34) et est installé dans une encoche (33) formée dans ladite tige (36), empêchant ainsi ledit dispositif de sollicitation (38) d'expanser et de déplacer ledit piston (32).

4. Pousse-seringue (10) selon la revendication 1, dans lequel une force de sollicitation dudit dispositif de sollicitation (38) sur ledit piston (32) et un amortissement hydraulique dudit fluide hydraulique (42) fournissent une force de pompage presque linéaire.

5. Pousse-seringue (10) selon la revendication 2, dans lequel ledit boîtier (12) a une fenêtre (50) à travers laquelle on peut observer le déplacement dudit mécanisme d'entraînement (14).

6. Pousse-seringue (10) selon la revendication 5, dans lequel ladite fenêtre (50) expose une languette (52) formée sur ladite tige (36).
